# EUROPÄISCHE PATENTSCHRIFT

(11) **EP 4 363 407 B1**
(45) Veröffentlichungstag und Bekanntmachung des Hinweises auf die Patenterteilung: **16.04.2025**
(21) Anmeldenummer: 22737468.3
(22) Anmeldetag: 24.06.2022
(51) Int. Cl.: C07D 261/04

(54) **VERFAHREN ZUR HERSTELLUNG VON ISOXAZOLINCARBONSÄURE-DERIVATEN**
METHOD FOR MANUFACTURING ISOXAZOLINECARBOXYLIC ACID DERIVATIVES
PROCÉDÉ DE PRODUCTION DE DÉRIVÉS D'ACIDE CARBONIQUE ISOXAZOLINE

(30) Priorität: 29.06.2021 EP 21182458
(43) Veröffentlichungstag der Anmeldung: 08.05.2024
(73) Patentinhaber: Bayer Aktiengesellschaft, 51373 Leverkusen (DE)
(72) Erfinder: LISHCHYNSKYI, Anton, 51373 Leverkusen (DE); MEMMEL, Frank, 51373 Leverkusen (DE); FORD, Mark James, 51373 Leverkusen (DE); HAAF, Klaus Bernhard, 51373 Leverkusen (DE)
(74) Vertreter: BIP Patents
(86) Internationale Anmeldenummer: PCT/EP2022/067334
(87) Internationale Veröffentlichungsnummer: WO 2023/274870

(56) Entgegenhaltungen:
- KANEMASA S ET AL: "Lewis Acid Coordinated Nitrile Oxide and Nitrile Imine 1,3-Dipoles. syn-Selective Cycloadditions to 2-(1-Hydroxyalkyl)acrylates", BULLETIN OF THE CHEMICAL SOCIETY OF JAPAN, CHEMICAL SOCIETY OF JAPAN,NIPPON KAGAKUKAI, JP, vol. 66, no. 9, 1 September 1993 (1993-09-01), pages 2685 - 2693, XP008121181, ISSN: 0009-2673, DOI: 10.1246/BCSJ.66.2685
- MICÚCH PETER ET AL: "Reversal of Diastereoselectivity of Nitrile Oxide 1,3-Dipolar Cycloadditions by Mg(II). Acceleration of Cycloaddition by Microwave Irradiation", TETRAHEDRON, vol. 56, no. 30, 2000, pages 5465 - 5472, XP085022593, ISSN: 0040-4020, DOI: 10.1016/S0040-4020(00)00454-3
- ANNUNZIATA RITA ET AL: "1,3-Dipolar Cycloadditions to Baylis-Hillman Adducts: Rationale for the Observed Diastereoselectivity", THE JOURNAL OF ORGANIC CHEMISTRY, vol. 60, no. 15, 1 July 1995 (1995-07-01), pages 4697 - 4706, XP055863137, ISSN: 0022-3263, DOI: 10.1021/jo00120a009

## Beschreibung

Die vorliegende Erfindung betrifft ein neues Verfahren zur Herstellung von Isoxazolincarbonsäure-Derivaten der Formel (**I**).

Isoxazolincarbonsäure-Derivate der allgemeinen Formel (**I**) sind wichtige Vorstufen von agrochemischen Wirkstoffen (vgl. WO 2018/228985).

Im Stand der Technik sind zahlreiche Zyklisierungsmethoden zur Herstellung von Isoxazolincarbonsäure-Derivaten beschrieben, beispielsweise Tetrahedron Letters, 1991, 6367 - 6370; Eur. J. Org. Chem. 2008, 5446 - 5460; Bull. Chem. Soc. Jpn. 1993, 2685. Es werden mögliche Übergangszustände für die Cycloaddition diskutiert. Außerdem werden Ausbeuten und Isomerenverhältnisse in Abhängigkeit von den Reaktionsbedingungen gezeigt.

Werden die Verbindungen der vorliegenden Erfindung nach einem der literaturbekannten Verfahren durchgeführt, resultieren Ausbeuten und Isomerenreinheiten, die für eine großtechnische Synthese nicht ausreichend sind.

Der Erfindung lag somit die Aufgabe zugrunde, ein Verfahren zur Herstellung von Isoxazolincarbonsäure-Derivaten der allgemeinen Formel (**I**) zur Verfügung zu stellen, welches für die großtechnische Synthese geeignet ist sowie eine hohe Ausbeute und Isomerenreinheit aufweist, sodass aufwändige Reinigungsmethoden entfallen können.

Die Aufgabe wurde erfindungsgemäß gelöst durch ein Verfahren zur Herstellung von Isoxazolincarbonsäure-Derivaten der Formel (I) 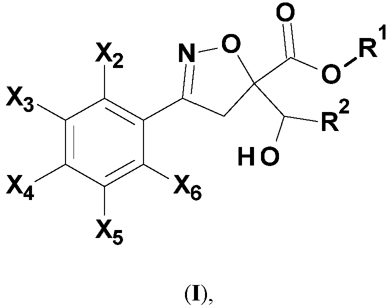 worin
- X²: H, C₁-C₄-Alkyl, C₁-C₄-Fluoralkyl, C₁-C₄-Fluoralkoxy, C₁-C₄-Alkoxy, Fluor, CN ist,
- X³: H, C₁-C₄-Alkyl, C₁-C₄-Fluoralkyl, C₁-C₄-Fluoralkoxy, C₁-C₄-Alkoxy, Fluor, Chlor, CN ist,
- X⁴: H, C₁-C₄-Alkyl, C₁-C₄-Fluoralkyl, C₁-C₄-Fluoralkoxy, C₁-C₄-Alkoxy, Fluor, CN ist,
- X⁵: H, C₁-C₄-Alkyl, C₁-C₄-Fluoralkyl, C₁-C₄-Fluoralkoxy, C₁-C₄-Alkoxy, Fluor, Chlor, CN ist,
- X⁶: H, C₁-C₄-Alkyl, C₁-C₄-Fluoralkyl, C₁-C₄-Fluoralkoxy, C₁-C₄-Alkoxy, Fluor, CN ist,
- R¹: H, C₁-C₄-Alkyl ist,
- R²: C₁-C₄-Alkyl ist,
dadurch gekennzeichnet, dass die Verbindungen der allgemeinen Formel (**II**) 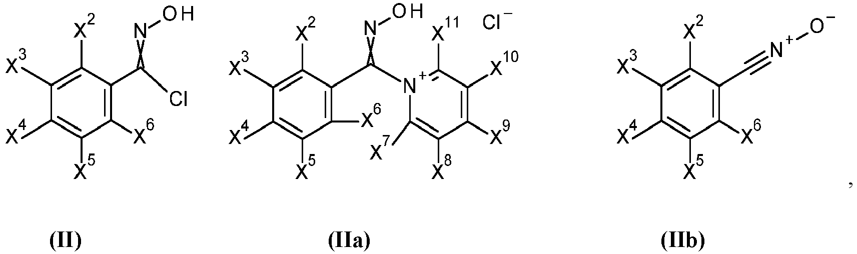 worin
X² bis X⁶ die oben genannten Bedeutungen hat,
X⁷, X⁸, X¹⁰, X¹¹ unabhängig voneinander H oder C₁-C₄-Alkyl bedeuten,
X⁹ H, C₁-C₄-Alkyl oder N(C₁-C₄-Alkyl)₂ bedeuten,
mit Verbindungen der Formel (**III**) 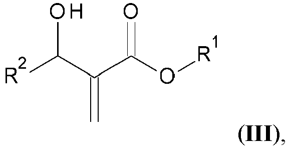
worin
   - R¹ und R²: die oben genannten Bedeutungen haben,
unter Zugabe einer Reagenzienkombination, die es ermöglicht, dass - bezogen auf Verbindungen der allgemeinen Formel (**II**) - 2,0 bis 4,5 Äquivalente einer reaktiven Spezies "R³OMgHal" (**IV**), gebildet werden können, wobei
   - R³: C₂-C₆-Alkyl, unsubstituiertes oder mit C₁-C₆-Alkyl substituiertes Benzyl bedeutet und
   - Hal: für Halogen steht,
zu Verbindungen der allgemeinen Formel (**I**) umgesetzt werden.

Bevorzugte Restedefinitionen für die Verbindungen der allgemeinen Formeln (**I**), (**II**) und (**III**) sind die folgenden:
- X²: ist H, Methyl, Trifluormethyl, Difluormethyl, Difluormethoxy, Trifluormethoxy, Fluor, Methoxy, CN,
- X³: ist H, Methyl, Trifluormethyl, Difluormethyl, Difluormethoxy, Trifluormethoxy, Fluor, Chlor, Methoxy, CN,
- X⁴: ist H, Methyl, Trifluormethyl, Difluormethyl, Difluormethoxy, Trifluormethoxy, Fluor, Methoxy, CN,
- X⁵: ist H, Methyl, Trifluormethyl, Difluormethyl, Difluormethoxy, Trifluormethoxy, Fluor, Chlor, Methoxy, CN,
- X⁶: ist H, Methyl, Trifluormethyl, Difluormethyl, Difluormethoxy, Trifluormethoxy, Fluor, Methoxy, CN,
- X⁷, X⁸, X¹⁰, X¹¹: bedeuten unabhängig voneinander H, Methyl, Ethyl,
- X⁹: bedeutet H, Methyl, Ethyl oder N(Methyl)₂
- R¹: ist H, Methyl, Ethyl, i-Propyl, i-Butyl,
- R²: ist Methyl, Ethyl.

Bevorzugt wird die Verbindung der allgemeinen Formeln (IV) mit einer der folgenden Reagenzienkombinationen generiert:
- R⁴MgHal und R³OH oder
- MgHal₂ und R³OM oder
- MgHal₂ und Mg(OR³)₂, wobei
   - R³: C₂-C₆-Alkyl, Benzyl, Methylbenzyl bedeutet,
   - Hal: Halogen bedeutet,
   - M: Alkalimetall bedeutet,
   - R⁴: C₁-C₆-Alkyl, Aryl, Benzyl, Allyl, Vinyl bedeutet.

Alternativ wird die Verbindung der allgemeinen Formeln (IV) mit der folgenden Reagenzienkombination generiert:
R⁵MgHal und R⁶R⁷CO, wobei
- R⁵: C₁-C₆-Alkyl, Aryl, Benzyl bedeutet,
- R⁶, R⁷: H, C₁-C₆-Alkyl, Aryl bedeutet, und die resultierende Restedefinition
- R³: R⁵R⁶R⁷C ist.

Bevorzugte Restedefinitionen für die Verbindungen der allgemeinen Formeln (**IV**), hergestellt über die alternative Reagenzienkombination:
R⁵MgHal und R⁶R⁷CO,
sind die folgenden:
   - R⁵: bedeutet C₁-C₄-Alkyl, Phenyl, Benzyl, p-Tolyl,
   - R⁶, R⁷: bedeutet H, C₁-C₄-Alkyl, Phenyl.

Besonders bevorzugte Restedefinitionen für die Verbindungen der allgemeinen Formeln (I), (II) und (III) sind die folgenden:
- X²: ist H,
- X³: ist H, Methyl, Trifluormethyl, Difluormethyl, Fluor, Chlor, Methoxy, CN,
- X⁴: ist Fluor, H,
- X⁵: ist H, Methyl, Trifluormethyl, Difluormethyl, Fluor, Chlor, Methoxy, CN,
- X⁶: ist H,
- X⁷, X⁸, X¹¹: bedeuten unabhängig voneinander H, Methyl, Ethyl,
- X⁹: ist H, N(Methyl)₂
- X¹⁰: ist H,
- R¹: ist H, Methyl, i-Propyl, i-Butyl.
- R²: ist Methyl.

Besonders bevorzugt wird die Verbindung der allgemeinen Formeln (**IV**) mit einer der folgenden Reagenzienkombinationen generiert:
- R⁴MgHal und R³OH oder
- MgHal₂ und R³OM oder
- MgHal₂ und Mg(OR³)₂, wobei

- R³: C₂-C₄-Alkyl bedeutet,
- Hal: Brom, Chlor bedeutet,
- M: Alkalimetall bedeutet,
- R⁴: C₁-C₄-Alkyl, Phenyl, Benzyl, p-Tolyl, Vinyl bedeutet.

Ganz besonders bevorzugte Restedefinitionen für die Verbindungen der allgemeinen Formeln (**I**), (**II**) und (**III**) sind die folgenden:
- X²: ist H,
- X³: ist H, Fluor,
- X⁴: ist H, Fluor,
- X⁵: ist H, Fluor,
- X⁶: ist H,
- X⁸: ist H, Methyl, Ethyl
- X⁷, X¹¹: ist unabhängig voneinander H, Methyl,
- X⁹, X¹⁰: ist H
- R¹: ist H, Methyl, *i*-Butyl.
- R²: ist Methyl.

Ganz besonders bevorzugt wird die Verbindung der allgemeinen Formeln (**IV**) mit einer der folgenden Reagenzienkombinationen generiert:
- R⁴MgHal und R³OH oder
- MgHal₂ und R³OM oder
- MgHal₂ und Mg(OR³)₂, wobei

- R³: *i*-Propyl, *i*-Butyl bedeutet,
- Hal: Brom, Chlor bedeutet,
- M: Natrium bedeutet,
- R⁴: Methyl, Ethyl, *n*-Butyl, *i*-Propyl bedeutet.

Am stärksten bevorzugte Restedefinitionen für die Verbindungen der allgemeinen Formeln (**I**), (**II**) und (**III**) sind die folgenden:
- X²: ist H,
- X³: ist Fluor,
- X⁴: ist H,
- X⁵: ist Fluor,
- X⁶: ist H,
- X⁷, X³, X¹¹: ist unabhängig voneinander H, Methyl,
- X⁹, X¹⁰: ist H,
- R¹: ist H, Methyl,
- R²: ist Methyl.

Zum Teil aus dem Stand der Technik bekannt sind Verbindungen der Formel (**I**), 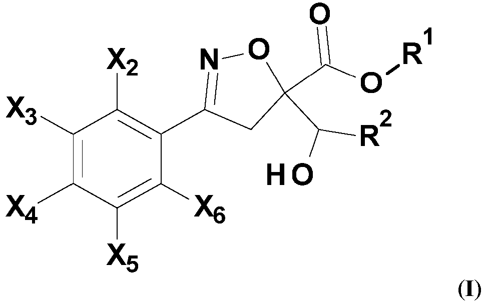
wobei die Reste den oben genannten allgemeinen, bevorzugten, besonders bevorzugten, ganz besonders bevorzugten und am stärksten bevorzugten Definitionen entsprechen
und als Isomerengemische vorliegen:

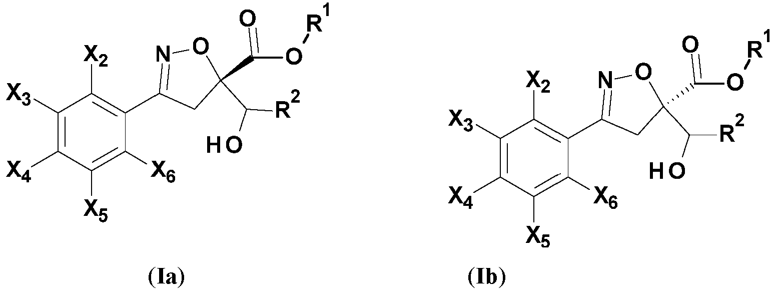

Das Isomerenverhältnis zwischen (**Ia**) und (**Ib**) variiert, im Allgemeinen liegt (**Ia**) im Überschuss vor. Dieser gewünschte Überschuss wird durch die optimierten Reaktionsbedingungen gegenüber dem Stand der Technik gesteigert. Für das Erzielen eines hohen Diastereomerenüberschusses ist insbesondere vorteilhaft, wenn 2,0 bis 4,5 Äquivalente einer reaktiven Spezies "R³OMgHal" (**IV**) im Reaktionsgemisch vorliegen.

Die Verbindungen der Formel **(I)** können als entsprechende Ester oder als Carbonsäure nach einer hydrolytischen Aufarbeitung isoliert werden.

Das d.r. Verhältnis bis 100:0 kann bei den Verbindungen der Formel **(I)** erreicht werden über eine Anreicherung durch Kristallisation.

Ein weiterer Gegenstand der vorliegenden Erfindung sind Verbindungen der Formel (**Ia**) und (**Ib**), in einem beliebigen Mischungsverhältnis, ausgenommen 1: 1, 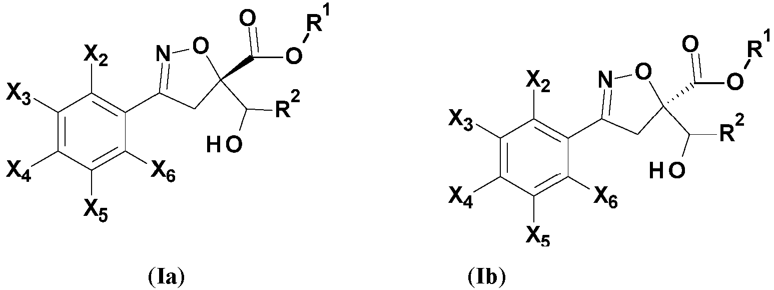 wobei die Reste die folgenden sind: X² ist H, X³ ist Fluor, X⁴ ist H, X⁵ ist Fluor, X⁶ ist H, R¹ ist H, Methyl und R² ist Methyl; ausgenommen 3-(3,5-Difluorphenyl)-5-(1-hydroxyethyl)-4H-isoxazol-5-carbonsäuremethylester.

Bevorzugt ist das Mischungsverhältnis (**Ia**): (**Ib**) von mindestens 90:1, besonders bevorzugt von mindestens 95:5, ganz besonders bevorzugt von mindestens 99:1.

Wird als Edukt enantiomerenreines (*S*)-(**III**) verwendet, lassen sich die Verbindungen der Formel (**Iaa**) und (**Iba**) herstellen.

Ein weiterer Gegenstand der vorliegenden Erfindung sind Verbindungen der Formel (**Iaa**) und (b), in einem beliebigen Mischungsverhältnis, ausgenommen 1:1, 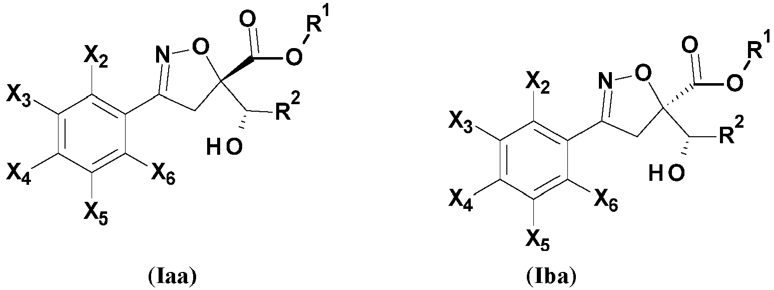 wobei die Reste die folgenden sind: X² ist H, X³ ist Fluor, X⁴ ist H, X⁵ ist Fluor, X⁶ ist H, R¹ ist H, Methyl und R² ist Methyl; ausgenommen 3-(3,5-Difluorphenyl)-5-(1-hydroxyethyl)-4H-isoxazol-5-carbonsäuremethylester.

Bevorzugt ist das Mischungsverhältnis (**Iaa**): (**Iba**) von mindestens 90:1, besonders bevorzugt von mindestens 95:5, ganz besonders bevorzugt von mindestens 99:1.

Wird als Edukt enantiomerenreines (*R*)-(**III**) verwendet, lassen sich die Verbindungen der Formel (**Iab**) und (**Ibb**) herstellen.

Ein weiterer Gegenstand der vorliegenden Erfindung sind Verbindungen der Formel (**Iab**) und (**Ibb**), in einem beliebigen Mischungsverhältnis, ausgenommen 1: 1, 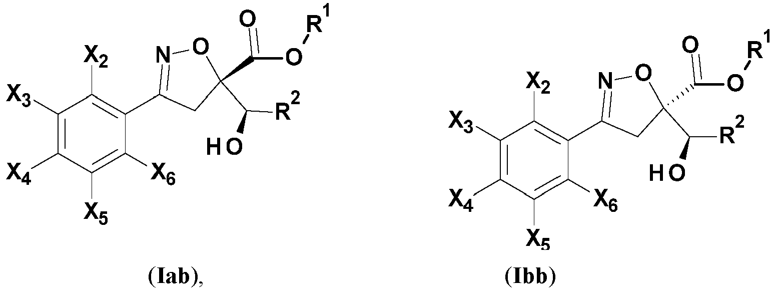 wobei die Reste die folgenden sind: X² ist H, X³ ist Fluor, X⁴ ist H, X⁵ ist Fluor, X⁶ ist H, R¹ ist H, Methyl und R² ist Methyl; ausgenommen 3-(3,5-Difluorphenyl)-5-(1-hydroxyethyl)-4H-isoxazol-5-carbonsäuremethylester und 3-Phenyl-5-(1-hydroxyethyl)-4H-isoxazol-5-carbonsäuremethylester.

Bevorzugt ist das Mischungsverhältnis (**Iab**): (**Ibb**) von mindestens 90:1, besonders bevorzugt von mindestens 95:5, ganz besonders bevorzugt von mindestens 99:1.

### Erläuterung der Verfahren und Zwischenprodukt

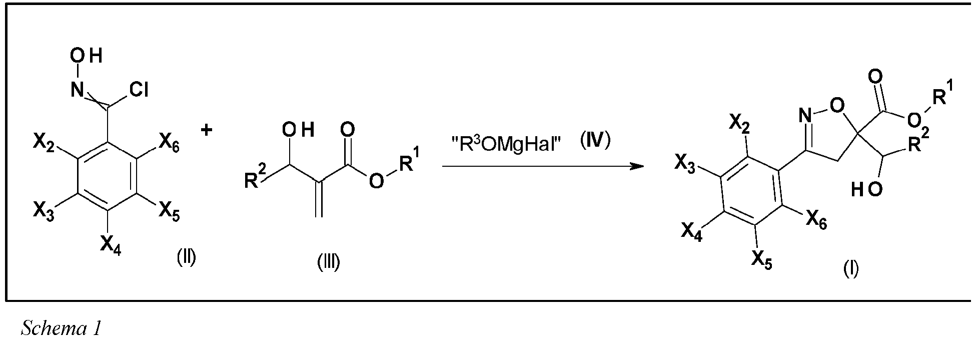

Die Aufgabe wurde gelöst durch ein Verfahren zur Herstellung von Isoxazolincarbonsäure-Derivaten der Formel (**I**) dadurch gekennzeichnet, dass die Verbindungen der allgemeinen Formel (**II**) mit Verbindungen der Formel (**III**) unter Zugabe einer Reagenzienkombination, die es ermöglicht, dass 2,0 bis 4,5 Äquivalente einer reaktiven Spezies "R³OMgHal" (**IV**) bezogen auf Verbindungen der allgemeinen Formel (**II**) gebildet werden können, zu Verbindungen der allgemeinen Formel (**I**) umgesetzt werden (Schema 1).

Der Tabelle 1 können verschiedene mögliche Reagenzienkombinationen entnommen werden, wobei diese Auswahl nicht abschließend ist.

Bevorzugt sind 2,8 bis 3,2 Äquivalente der reaktiven Spezies "R³OMgHal" (**IV**) bezogen auf Verbindungen der allgemeinen Formel (**II**).

Besonders bevorzugt sind 3 Äquivalente der reaktiven Spezies "R³OMgHal" (**IV**) bezogen auf Verbindungen der allgemeinen Formel (**II**).

Die Zyklisierung wird gewöhnlich in einem Temperaturbereich von -25°C bis 70°C, vorzugsweise 10°C bis 30 °C durchgeführt.

Des Weiteren wird die Zyklisierung gegebenenfalls in Gegenwart eines Lösungs- oder Verdünnungsmittels oder eines Lösungsmittelgemisches durchgeführt. Bevorzugt sind die Lösungsmittel Toluol, Xylol, Tetrahydrofuran (THF), Isopropylacetat (*i*-PrOAc), Acetonitril, Methyl-tert-butylether (MTBE), Methyl-THF, Ethylacetat (EtOAc) oder Mischungen in beliebigen Verhältnissen daraus.

Die Verbindungen der allgemeinen Formel (**III**) werden über ein zweistufiges Verfahren hergestellt, und sind literaturbekannt. Die erste Stufe ist eine Baylis-Hillman Reaktion. Eine relevante Literaturstelle ist: Drewes, S. E.; Hoole, R. F. A. [Synthetic Communications, 1985, vol. 15, 12, p. 1067 - 1074]. Für die zweite Stufe ist eine relevante Literaturstelle: Nascimento et al. (2003, Tetrahedron Asymmetry 14, 311-311).

Die Verbindungen der allgemeinen Formel (**II**) und (**III**) sind des Weiteren bekannt aus WO 2018/228985. Die Herstellung der Verbindungen der Formel (**IIa**) aus (**II**) ist aus Binenfeld, Zlatko; et al Glasnik Hemijskog Drustva Beograd (1966), 31(4-6), 243-50 und Daroszewski, J.; et al Pharmazie (1986), 41(10), 699-702 bekannt.

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR Spektroskopie und/oder LC/MS (Liquid Chromatography Mass Spectrometry) charakterisiert.

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

### Beispiel 1

2-Propanol (48 mL) und THF (61 mL) werden in einem 1 L Reaktionsgefäß bei 24°C unter Argon Atmosphäre vorgelegt. Das Gemisch wird auf 0°C abgekühlt. Dann werden 204 mL einer Methylmagnesiumchloridlösung (3M in THF) hinzugegeben, und zwar über zwei Stunden, wobei die Reaktionstemperatur bei 10 bis 25 °C gehalten wird (Kühlung mit dem Eisbad). Methan entweicht und ein weißer Feststoff fällt aus. Die entstehende Suspension wird weiter gerührt und auf 20°C erwärmt, nachdem die Gasentwicklung beendet ist. Dann werden 27,46 g Methyl-3-hydroxy-2-methylene-bu-tanoat bei 20°C zur Reaktionsmischung über 30 Minuten hinzugefügt. Dabei wird die Reaktionstemperatur mit einem Wasserbad bei 18 bis 22 °C gehalten. Nach der Zugabe wir das Reaktionsgemisch noch für 10 Minuten weiter gerührt, bevor dann bei 20°C 240 g 3,5-Difluor-N-hydroxybenzencarboximidoylchlorid in einem Lösungsmittelgemisch aus Toluol/THF/i-PrOAc/DBF (16.3 Gew.-%) hinzugegeben werden, wobei das Reaktionsgefäß mit einem Eisbad gekühlt wird. Nach vollständiger Zugabe wird das Reaktionsgemisch aufgearbeitet, und zwar dadurch, dass das Reaktionsgemisch bei Raumtemperatur auf 40,25 g HCl (37 Gew.-%) in 83,85 g Wasser gegeben wird. Die organische Phase wird abgetrennt. Es wird mit Toluol (25 mL) nachgespült und destilliert (48-58 °C Kopftemperatur, 580 bis 160 mbar).

Die Hydrolyse erfolgt mit 69,4 g NaOH (20 Gew.-%, 1.7 Äquivalente). Die Lösung wird 1 Stunde lang bei 60°C gerührt bis sie vollendet ist.

Zur Aufarbeitung werden 100 mL Toluol, 100 mL Wasser und 3,00 g H₂SO₄ (20 Gew.-%) zum Reaktionsgemisch zugegeben und es bildeten sich zwei Phasen. Die organische Phase wird abgetrennt und die Wasserphase (pH~7) mit 50 mL Toluol gewaschen, um Reste von DBF zu extrahieren. Zu der resultierenden Wasserphase, die das Natrium-Salz des Produkts enthält, werden 200 mL i-PrOAc zugegeben und anschließend bei Raumtemperatur und unter kräftigem Rühren 65,09 g H₂SO₄ (20 Gew.-%), was zu einem Zweiphasengemisch mit dem pH-Wert 1 der Wasserphase führt. Die organische Phase wird abgetrennt und die Wasserphase wird mit 50 mL i-PrOAc extrahiert, um den Rest des Produkts zu entfernen. Die i-PrOAc-Phasen werden vereinigt und unter Rühren unter vermindertem Druck (210 bis 200 mbar) bei 50 °C destilliert, was zu einer dicken, aber noch gut rührbaren Suspension führte (nach Destillation von ca. 150 mL i-PrOAc). Dann werden 100 mL Toluol zugegeben und die Destillation unter Rühren bei vermindertem Druck (200 bis 150 mbar) bei 50°C fortgesetzt (ca. 100 mL i-PrOAc/Toluol-Gemisch wurden abdestilliert). Das Gemisch wird auf Raumtemperatur abgekühlt und über Nacht zur Vervollständigung der Kristallisation stehen gelassen. Die Suspension wird dann filtriert und auf dem Filter mit weiteren 50 mL Toluol gewaschen, was zu einem Feststoff führte, der im Vakuumtrockenschrank (40°C, 20 mbar) getrocknet wurde, was zu 45,54 g (98,0%-ig, 80,6 % Ausbeute) des Produkts führte. Das Produkt (5S)-3-(3,5-Difluorphenyl)-5-[(1S)-1-hydroxyethyl]-4,5-dihydro-1,2-oxazol-5-carbonsäure wurde in der HPLC analysiert und zeigte einen d.r. von 97,6:2,4. Die Mutterlauge zeigte einen d.r. von (31,3:68,7).

¹H-NMR (400MHz, DMSO-*d₆*): δ (ppm) = 1.12 (d, *J =* 6.5 Hz, 3H), 3.62 (d, *J =* 17.8, 1H), 3.68 (d, *J =* 17.8, 1H), 4.10 (q, *J* = 6.4 Hz, 1H), 5.30 (bs, 1H), 7.33-7.48 (m, 3H), 13.24 (bs, 1H).

¹⁹F-NMR (376MHz, DMSO-*d₆*): δ (ppm) = -108.7 (m, 2F).

### Beispiel 2

In einem 2 L Doppelmantelgefäß werden 74,74 g Magnesium (3,075 Äquivalente) in THF (1 L), unter Stickstoff Atmosphäre und bei 20°C Manteltemperatur, vorgelegt. Anschließend werden 2,5 mL 2-Propylmagnesiumchloridlösung (2M in THF, 0,005 Äquivalente) zugegeben und 10 min nachgerührt, dann werden 248,00 g 2-Chlorpropan (99%, 3,126 Äquivalente) langsam zugetropft. Die Innentemperatur steigt auf 35°C und die Manteltemperatur wird auf 10°C eingestellt. Innerhalb von 2 Stunden wird das gesamte 2-Chlorpropan zugetropft. Die Innentemperatur wird durch Kühlung bei ca. 30 bis 34°C gehalten. Das Magnesium reagiert langsam ab. Nach dem Ende der Zugabe fällt die Temperatur langsam und die Manteltemperatur wird langsam auf 30°C erhöht. Insgesamt wird zwei Stunden weiter gerührt, und zwar bei 29 bis 33°C Innentemperatur, bis nur noch wenig Magnesiumflocken vorhanden sind. 185,17 g 2-Propanol (3,075 Äquivalente) werden bei 15°C Manteltemperatur zugetropft. Die Innentemperatur wird bei 30 bis 35°C gehalten. Stetige Gasentwicklung (Propan) wird beobachtet. Ein Feststoff fällt langsam aus. Es entsteht eine dicke noch rührbare Suspension. Nach der Zugabe über 1 Stunde und 30 min liegt eine dicke, graue Suspension vor. Der Ansatz wird auf 20°C abgekühlt und 130,40 g Methyl-(3S)-3-hydroxy-2-methylenbutanoat (1 Äquivalent) zugegeben. Es wird 10 min nachgerührt und die Manteltemperatur wird auf 10°C gestellt. Danach wird eine Lösung von 3,5-Difluor-N-hydroxybenzencarboximidoylchlorid (15,2 %, 1260,30 g) bei 18 bis 20°C Innentemperatur und 10°C Manteltemperatur über 1,5 Stunde zugetropft. Nach dem Ende der Zugabe wird 1 Stunde bei 20°C nachgerührt.

Für die Aufarbeitung wird die Reaktionsmischung auf 403 g Eis und 200 g HCl-Lösung (37 Gew.-%) gegeben. Die organische Phase wird abgetrennt und bei 50°C Badtemperatur und 350 mbar eingeengt. Zu dem erhaltenen Rückstand werden 314,40 g Natronlauge (20 Gew.-%, 1,57 Äquivalente) zugegeben und zwei Stunden bei 60°C gerührt, bis die Hydrolyse vollendet ist. Bei 325 mbar und 60°C Badtemperatur wird das Lösungsmittel abdestilliert. Zum Rückstand werden 450 g Wasser und 372 g Toluol zugegeben. Nach der Extraktion wird die Toluolphase abgetrennt.

Die Wasserphase wird dann nach Ansäuern mit Schwefelsäure (500 g, 20 Gew.-%) mit i-Propylacetat extrahiert. Das Lösungsmittel der organischen Phase wird entfernt (40 bis 61 °C Innentemperatur, 200 mbar). Die resultierende Suspension wird bei 20 °C filtriert und auf dem Filter mit weiteren 280 mL Toluol gewaschen, was zu einem Feststoff führte, der an der Luft getrocknet war.

Das erhaltene Produkt ist (5S)-3-(3,5-Difluorphenyl)-5-[(1S)-1-hydroxyethyl]-4,5-dihydro-1,2-oxazol-5-carbonsäure: 208,5 g (98,1%-ig, d.r. >99,9:0,1, 75,4% Ausbeute).

¹H-NMR (401MHz, DMSO-*d₆*): δ (ppm) = 1.11 (d, *J =* 6.5 Hz, 3H), 3.61 (d, *J =* 17.8, 1H), 3.67 (d, *J =* 17.8, 1H), 4.10 (q, *J =* 6.4 Hz, 1H), 5.20 (bs, 1H), 7.34-7.45 (m, 3H), 13.28 (bs, 1H).

¹⁹F-NMR (376MHz, DMSO-*d₆*): δ (ppm) = -108.7 (m, 2F).

### Beispiel 10

6.79g (51.2 mmol) Methyl (3S)-3-hydroxy-2-methylene-butanoate wurden unter Argonatmosphäre bei Raumtemperatur in 650 ml Dichlormethan gelöst und mit Isopropanol versetzt. Die klare Lösung wurde auf 0°C gekühlt. Anschließend wurden 156 ml (156 mmol) EtMgBr (1M) in THF langsam (exotherm) zugetropft. Die Lösung wurde trüb. Dann wurde eine Lösung vom 10.00 g (52.2 mmol) 3,5-Difluoro-N-hydroxy-benzimidoyl chlorid in 100 ml DCM langsam unter Rühren zugetropft (15 min) und langsam auf Raumtemperatur erwärmt. Es trat eine deutliche Gelbfärbung auf. DC in EE / nHeptan 1: 1 zeigte vollständigen Umsatz nach 30 min.

Aufarbeitung:
Die Lösung wurde auf 1 l eines 1:1 Gemisches von 2N HCl und gesättigter NaCl Lösung gegeben und zweimal mit je 400 ml Methylenchlorid extrahiert, über Na₂SO₄ getrocknet, filtriert und eingeengt. Die LCMS Analyse des Rohproduktes zeigte ein Diastereomerenverhältnis der Produkte von 86% zu 14%.
Chromatographie über Kieselgel (nHep/Essigester)
Fr. 1 m = 600 mg (4.0 %) lipophiles Diastereomer

¹H NMR (CDCl₃) : 1.10 (d, 3H, CHCH₃), 2.3 (s br., 1H, OH), 3.53 (d, 1H, CHH isoxazolin), 3.72 (d, 1H, CHH isoxazolin), 3.84b(s, 3H, OCH₃), 4,34 (q, 1H, CHCH₃), 6.88 (tt, 1H, arom.H), 7.22 (m, 2H, arom. H).

Fr. 2 m = 7700 mg (51.7%) polarers Diastereomer
¹H NMR (CDCl₃) : 1.29 (d, 3H, CHCH₃), 2.10 (d, 1H, OH), 3.57 (d, 1H, CHH isoxazolin), 3.69 (d, 1H, CHH isoxazolin), 3.84b(s, 3H, OCH₃), 4,24 (q, 1H, CHCH₃), 6.88 (tt, 1H, arom.H), 7.18 (m, 2H, arom. H).

**Tabelle 1:**

| | **R1, (III) Stereochemie, Äquiv.** | **Reagenzienkombination (IV)** | **Lösungsmittel, Temperatur** | **Ausbeute** | **Diastereomerenverhältnis, d.r.** |
|---|---|---|---|---|---|
| Erfindungsgemäß | | | | | |
| Beispiel 1 | Methyl, rac, 1 Äq | 3,0 Äq MeMgCl (22% in THF) 3,05 Äq i-PrOH | 1. THF, 10-25 °C 2. THF/Toluene/i-PrOAc/DBF, 20 °C | 81% (isoliert) | 98:2 (isoliert) 93:7 (Reaktions mischung) |
| Beispiel 2 | Methyl, S, 1 Äq | 3,075 Äq Mg, 3,075 Äq i-PrOH, 0,5 mol% i-PrMgCl, 3,126 Äq i-PrCl | 1. THF, 30-35 °C; 1. 2. THF/Toluene/ DBF, 18-20 °C | 75% (isoliert) | 100:0 (isoliert) 93:7 (Reaktions mischung) |
| Beispiel 3 | Methyl, S, 1 Äq | 3,6 Äq MgCl₂ 3,5 Äq i-PrONa (25% in THF) | 2. THF, 20-50 °C 3. THF/Toluene/ i-PrOAc/DBF, 20 °C | 85% (HPLC) | 92:8 |
| Beispiel 4 | Methyl, rac, 1 Äq | 1,5 Äq MgCl₂ 1,5 Äq Mg(OPr-i)₂ | 1. CH₃CN, 20-80 °C; 2. Toluol/EtOAc/DBF, - 20-20 °C | 84% (HPLC) | 94:6 |
| Beispiel 5 | i-Propyl, rac, 1 Äq | 3,075 Äq Mg, 3,075 Äq i-PrOH, 0,5 mol% i-PrMgCl, 3,126 Äq i-PrCl | 1. THF, 22-40 °C; 2. THF/Toluene/DBF, 20 °C | 79% (HPLC) | 94:6 |
| Beispiel 6 | i-Butyl, rac, 1 Äq | 3,0 Äq MeMgCl (22% in THF) 3,075 Äq i-PrOH | 1. THF, 25-35 °C; 2. THF/Toluene/DBF, 20 °C | 84% (HPLC) | 94:6 |
| Beispiel 7 | i-Butyl, rac, 1 Äq | 3,0 Äq MeMgCl (22% in THF) 3,075 Äq i-BuOH | 1. THF, 25-35 °C; 2. THF/Toluene/DBF, 20 °C | 83% (HPLC) | 93:7 |
| Beispiel 8 | i-Butyl, rac, 1 Äq | 3,075 Äq Mg, 3,075 Äq i-PrOH, 0,5 mol% i-PrMgCl, 3,126 Äq i-PrCl | 1. THF, 22-40 °C; 2. THF/Toluene/DBF, 20 °C | 83% (HPLC) | 95:5 |
| Beispiel 9 | i-Butyl, rac, 1 Äq | 3,075 Äq Mg, 3,075 Äq i-BuOH, 0,5 mol% i-PrMgCl, 3,126 Äq i-PrCl | 1. THF, 22-40 °C; 2. THF/Toluene/DBF, 20 °C | 84% (HPLC) | 93:7 |
| Beispiel 10 | Methyl, rac, 1 Äq | 3,0 Äq MeMgCl (22% in THF), 3,075 Äq CH₃CHO | 1. THF, -20 °C bis R.T. 2. THF/Toluene, 15-20 °C | 75% (HPLC) | 91:9 |

| **Bedingungen aus dem Stand der Technik angewendet auf die erfindungsgemäße Reaktion (Beispiel 10)** | | | | | |
|---|---|---|---|---|---|
| Carreira ACIE 2001, 40, 2082, JACS 2001, 123, 3611, OL 2007, 9, 3857 | Zyklisc he und azyklis che Allylal kohole | 3.3 Äq i-PrOH, 3 Äq EtMgBr | CH₂Cl₂, 0 °C - RT | 70% | 86:14 |

| **Stand der Technik (X₂₋₆=H)** | | | | | |
|---|---|---|---|---|---|
| Bull. Chem. Soc. Jpn. 1993, 2685-2689 | Methyl, 2 Äq | 2 Äq EtMgBr | CH₂Cl₂, -78 bis -30 °C /bis R.T. | 100 | 92:8 /89:11 |
| | Methyl, 1 Äq | 1 Äq NEt₃ | CH₂Cl₂, -30 °C | 90 | 30:70 |
| | Methyl, 1 Äq | 1 Äq EtMgBr | THF / CH₂Cl₂, -78 bis -30 °C | 93 / 86 | 63:37 / 81:19 |

## Patentansprüche

1. Verfahren zur Herstellung von Isoxazolincarbonsäure-Derivaten der Formel (**I**) 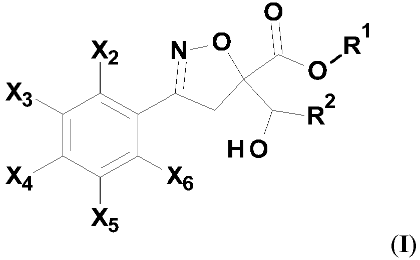 worin
X² H, C₁-C₄-Alkyl, C₁-C₄-Fluoralkyl, C₁-C₄-Fluoralkoxy, C₁-C₄-Alkoxy, Fluor, CN ist,
X³ H, C₁-C₄-Alkyl, C₁-C₄-Fluoralkyl, C₁-C₄-Fluoralkoxy, C₁-C₄-Alkoxy, Fluor, Chlor, CN ist,
X⁴ H, C₁-C₄-Alkyl, C₁-C₄-Fluoralkyl, C₁-C₄-Fluoralkoxy, C₁-C₄-Alkoxy, Fluor, CN ist,
X⁵ H, C₁-C₄-Alkyl, C₁-C₄-Fluoralkyl, C₁-C₄-Fluoralkoxy, C₁-C₄-Alkoxy, Fluor, Chlor, CN ist,
X⁶ H, C₁-C₄-Alkyl, C₁-C₄-Fluoralkyl, C₁-C₄-Fluoralkoxy, C₁-C₄-Alkoxy, Fluor, CN ist,
R¹ H, C₁-C₄-Alkyl ist,
R² C₁-C₄-Alkyl ist,
**dadurch gekennzeichnet, dass** die Verbindungen der allgemeinen Formel (**II**) 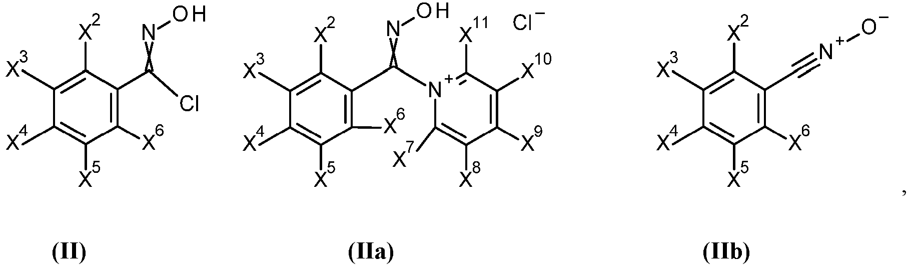 worin
X² bis X⁶ die oben genannten Bedeutungen hat,
X⁷, X⁸, X¹⁰, X¹¹ unabhängig voneinander H oder C₁-C₄-Alkyl bedeuten,
X⁹ H, C₁-C₄-Alkyl oder N(C₁-C₄-Alkyl)₂ bedeutet,
mit Verbindungen der Formel (**III**) 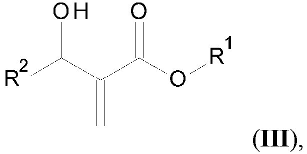
worin
R¹ und R² die oben genannten Bedeutungen haben,
unter Zugabe einer Reagenzienkombination, die es ermöglicht, dass - bezogen auf Verbindungen der allgemeinen Formel (**II**) - 2,0 bis 4,5 Äquivalente einer reaktiven Spezies "R³OMgHal" (**IV**), gebildet werden können, wobei
R³ C₂-C₆-Alkyl, unsubstituiertes oder mit C₁-C₆-Alkyl substituiertes Benzyl bedeutet und
Hal für Halogen steht,
zu Verbindungen der allgemeinen Formel (**I**) umgesetzt werden.

2. Verfahren nach Anspruch 1, wobei die Restedefinitionen für die Verbindungen der allgemeinen Formeln (**I**), (**II**) und (**III**) die folgenden sind:
X² ist H, Methyl, Trifluormethyl, Difluormethyl, Difluormethoxy, Trifluormethoxy, Fluor, Methoxy, CN,
X³ ist H, Methyl, Trifluormethyl, Difluormethyl, Difluormethoxy, Trifluormethoxy, Fluor, Chlor, Methoxy, CN,
X⁴ ist H, Methyl, Trifluormethyl, Difluormethyl, Difluormethoxy, Trifluormethoxy, Fluor, Methoxy, CN,
X⁵ ist H, Methyl, Trifluormethyl, Difluormethyl, Difluormethoxy, Trifluormethoxy, Fluor, Chlor, Methoxy, CN,
X⁶ ist H, Methyl, Trifluormethyl, Difluormethyl, Difluormethoxy, Trifluormethoxy, Fluor, Methoxy, CN,
R¹ ist H, Methyl, Ethyl, i-Propyl, i-Butyl,
R² ist Methyl, Ethyl.

3. Verfahren nach einem der Ansprüche 1 bis 2, wobei die Restedefinitionen für die Verbindungen der allgemeinen Formeln (**I**), (**II**) und (**III**) die folgenden sind:
X² ist H,
X³ ist H, Methyl, Trifluormethyl, Difluormethyl, Fluor, Chlor, Methoxy, CN,
X⁴ ist Fluor, H,
X⁵ ist H, Methyl, Trifluormethyl, Difluormethyl, Fluor, Chlor, Methoxy, CN,
X⁶ ist H,
R¹ ist H, Methyl, i-Propyl, i-Butyl.
R² ist Methyl.

4. Verfahren nach einem der Ansprüche 1 bis 3, wobei die Restedefinitionen für die Verbindungen der allgemeinen Formeln (**I**), (**II**) und (**III**) die folgenden sind:
X² ist H,
X³ ist H, Fluor,
X⁴ ist H, Fluor,
X⁵ ist H, Fluor,
X⁶ ist H,
R¹ ist H, Methyl, i-Butyl.
R² ist Methyl.

5. Verfahren nach einem der Ansprüche 1 bis 4, wobei die Restedefinitionen für die Verbindungen der allgemeinen Formeln (**I**), (**II**) und (**III**) die folgenden sind:
X² ist H,
X³ ist Fluor,
X⁴ ist H,
X⁵ ist Fluor,
X⁶ ist H,
R¹ ist H, Methyl.
R² ist Methyl.

6. Verfahren nach einem der Ansprüche 1 bis 5, wobei die Restedefinitionen für die Verbindungen der allgemeinen Formeln (**IIa**) die folgenden sind:
X⁷, X⁸, X¹⁰, X¹¹ bedeuten unabhängig voneinander H, Methyl, Ethyl,
X⁹ bedeutet H, Methyl, Ethyl, oder N(Methyl)₂.

7. Verfahren nach einem der Ansprüche 1 bis 6, wobei die Restedefinitionen für die Verbindungen der allgemeinen Formeln (**IIa**) die folgenden sind:
X⁷, X¹¹ ist H,
X⁸, X¹⁰ bedeuten unabhängig voneinander H, Methyl, Ethyl,
X⁹ ist H, Methyl, Ethyl, N(Methyl)₂.

8. Verfahren nach einem der Ansprüche 1 bis 7, wobei die Restedefinitionen für die Verbindungen der allgemeinen Formeln (**IIa**) die folgenden sind:
X⁸ ist H, Methyl, Ethyl,
X⁷, X¹¹ ist unabhängig voneinander H, Methyl,
X⁹, X¹⁰ ist H.

9. Verfahren nach einem der Ansprüche 1 bis 8, wobei die Verbindung der allgemeinen Formeln (**IV**) mit einer der folgenden Reagenzienkombinationen generiert werden:
- R⁴MgHal und R³OH oder
- MgHal₂ und R³OM oder
- MgHal₂ und Mg(OR³)₂, wobei
R³ C₂-C₆-Alkyl, Benzyl, Methylbenzyl bedeutet,
Hal Halogen bedeutet,
M Alkalimetall bedeutet,
R⁴ C₁-C₆-Alkyl, Aryl, Benzyl, Allyl, Vinyl bedeutet.

10. Verfahren nach einem der Ansprüche 1 bis 9, wobei die Verbindung der allgemeinen Formeln (IV) mit einer der folgenden Reagenzienkombinationen generiert werden:
- R⁴MgHal und R³OH oder
- MgHal₂ und R³OM oder
- MgHal₂ und Mg(OR³)₂, wobei
- R³ C₂-C₄-Alkyl bedeutet,
- Hal Brom oder Chlor bedeutet
- M Alkalimetall bedeutet,
- R⁴ C₁-C₄-Alkyl, Phenyl, Benzyl, p-Tolyl, Vinyl bedeutet.

11. Verfahren nach einem der Ansprüche 1 bis 10, wobei die Verbindung der allgemeinen Formeln (**IV**) mit einer der folgenden Reagenzienkombinationen generiert werden:
- R⁴MgHal und R³OH oder
- MgHal₂ und R³OM oder
- MgHal₂ und Mg(OR³)₂, wobei
R³ *i*-Propyl, *i*-Butyl bedeutet,
Hal Brom, Chlor bedeutet,
M Natrium bedeutet,
R⁴ Methyl, Ethyl, *n*-Butyl, *i*-Propyl bedeutet.

12. Verfahren nach einem der Ansprüche 1 bis 11, **dadurch gekennzeichnet, dass** 2,8 bis 3,2 Äquivalente der reaktiven Spezies "R³OMgHal" (**IV**) bezogen auf Verbindungen der allgemeinen Formel (**II**) verwendet werden.

13. Verfahren nach einem der Ansprüche 1 bis 12, **dadurch gekennzeichnet, dass** das Lösungsmittel Toluol, Xylol, Tetrahydrofuran (THF), Isopropylacetat (*i*-PrOAc), Acetonitril, Methyl-*tert*-butylether (MTBE), Methyl-THF, Ethylacetat (EtOAc) oder Mischungen in beliebigen Verhältnissen daraus ist.

14. Verfahren nach einem der Ansprüche 1 bis 13, **dadurch gekennzeichnet, dass** die Reaktion bei -25°C bis 70°C durchgeführt wird.

15. Verfahren nach einem der Ansprüche 1 bis 13, **dadurch gekennzeichnet, dass** die Reaktion bei 10°C bis 30 °C durchgeführt wird.

16. Verfahren nach einem der Ansprüche 1 bis 15, **dadurch gekennzeichnet, dass** das d.r. Verhältnis durch einen weiteren Kristallisationsschritt erhöht wird.

17. Verfahren nach einem der Ansprüche 1 bis 8, **dadurch gekennzeichnet, dass** die Verbindungen der allgemeinen Formel (**IV**) über eine Grignard-Reaktion generiert werden, und zwar mit der folgenden Reagenzienkombinationen:
R⁵MgHal und R⁶R⁷CO, wobei
R⁵ C₁-C₆-Alkyl, Aryl, Benzyl bedeutet,
R⁶, R⁷ H, C₁-C₆-Alkyl, Aryl bedeutet, und die resultierende Restedefinition
R³ R⁵R⁶R⁷C ist.

18. Verbindungen der Formel (**Ia**) und (**Ib**), in einem beliebigen Mischungsverhältnis, ausgenommen 1:1, wobei die Reste die Definitionen gemäß Anspruch 5 aufweisen, ausgenommen 3-(3,5-Difluorphenyl)-5-(1-hydroxyethyl)-4H-isoxazol-5-carbonsäuremethylester:

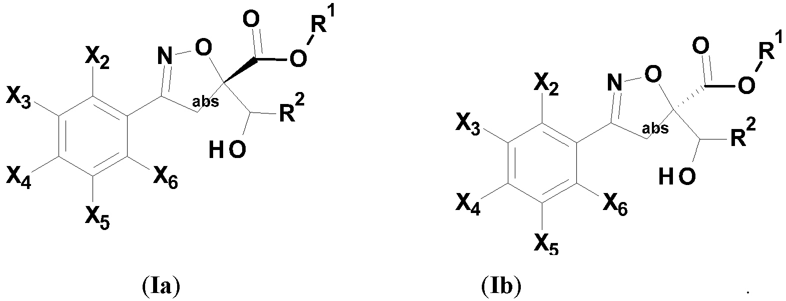

19. Verbindungen der Formel (**Iaa**) und (**Iba**), in einem beliebigen Mischungsverhältnis, ausgenommen 1:1, wobei die Reste die Definitionen gemäß Anspruch 5 aufweisen, ausgenommen 3-(3,5-Difluorphenyl)-5-(1-hydroxyethyl)-4H-isoxazol-5-carbonsäuremethylester:

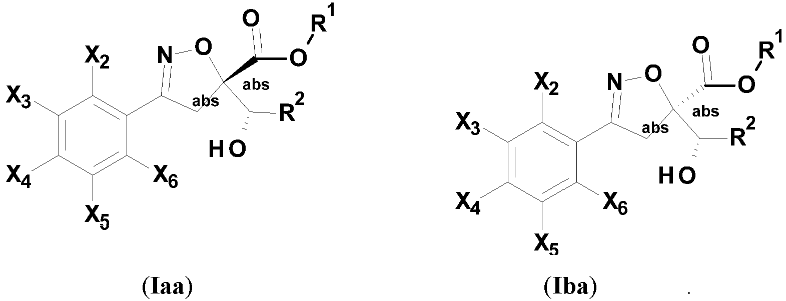

20. Verbindungen der Formel (**Iab**) und (**Ibb**), in einem beliebigen Mischungsverhältnis, ausgenommen 1:1, wobei die Reste die Definitionen gemäß Anspruch 5 aufweisen, ausgenommen 3-(3,5-Difluorphenyl)-5-(1-hydroxyethyl)-4H-isoxazol-5-carbonsäuremethylester:

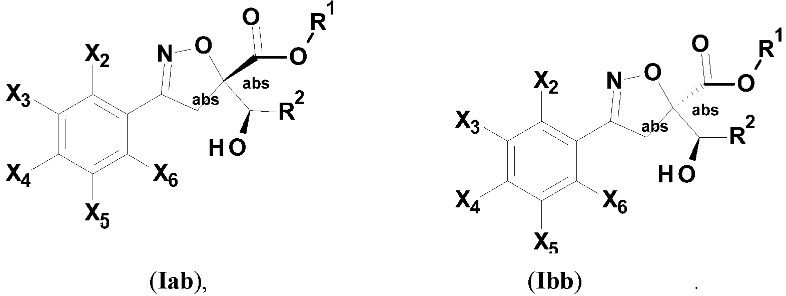

## Claims

1. Process for preparing isoxazolinecarboxylic acid derivatives of the formula (I) 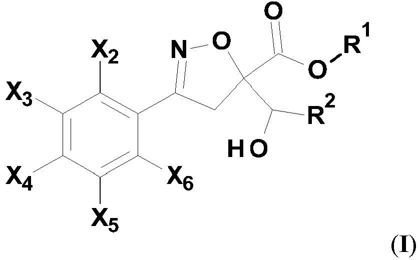 in which
X² is H, C₁-C₄-alkyl, C₁-C₄-fluoroalkyl, C₁-C₄-fluoroalkoxy, C₁-C₄-alkoxy, fluorine, CN,
X³ is H, C₁-C₄-alkyl, C₁-C₄-fluoroalkyl, C₁-C₄-fluoroalkoxy, C₁-C₄-alkoxy, fluorine, chlorine, CN,
X⁴ is H, C₁-C₄-alkyl, C₁-C₄-fluoroalkyl, C₁-C₄-fluoroalkoxy, C₁-C₄-alkoxy, fluorine, CN,
X⁵ is H, C₁-C₄-alkyl, C₁-C₄-fluoroalkyl, C₁-C₄-fluoroalkoxy, C₁-C₄-alkoxy, fluorine, chlorine, CN,
X⁶ is H, C₁-C₄-alkyl, C₁-C₄-fluoroalkyl, C₁-C₄-fluoroalkoxy, C₁-C₄-alkoxy, fluorine, CN,
R¹ is H, C₁-C₄-alkyl,
R² is C₁-C₄-alkyl,
**characterized in that** the compounds of the general formula (II) 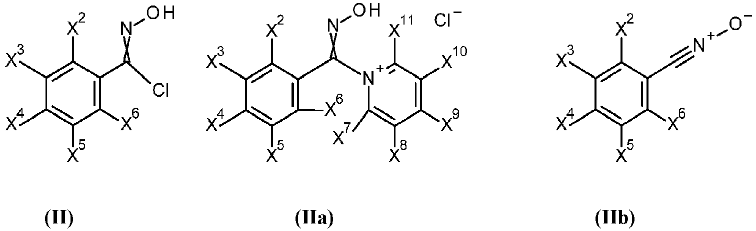
in which
X² to X⁶ have the definitions given above,
X⁷, X⁸, X¹⁰, X¹¹ are independently H or C₁-C₄-alkyl,
X⁹ is H, C₁-C₄-alkyl or N(C₁-C₄-alkyl)₂,
are reacted with compounds of the formula (III) 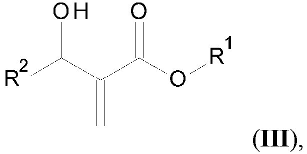
in which
R¹ and R² have the definitions given above,
with addition of a combination of reagents that enables formation of 2.0 to 4.5 equivalents of a reactive species "R³OMgHal" (IV) - based on compounds of the general formula (II), where
R³ is C₂-C₆-alkyl, unsubstituted or C₁-C₆-alkylsubstituted benzyl and
Hal is halogen,
to give compounds of the general formula (I).

2. Process according to Claim 1, wherein the definitions of the radicals in the compounds of the general formulae (I), (II) and (III) are as follows:
X² is H, methyl, trifluoromethyl, difluoromethyl, difluoromethoxy, trifluoromethoxy, fluorine, methoxy, CN,
X³ is H, methyl, trifluoromethyl, difluoromethyl, difluoromethoxy, trifluoromethoxy, fluorine, chlorine, methoxy, CN,
X⁴ is H, methyl, trifluoromethyl, difluoromethyl, difluoromethoxy, trifluoromethoxy, fluorine, methoxy, CN,
X⁵ is H, methyl, trifluoromethyl, difluoromethyl, difluoromethoxy, trifluoromethoxy, fluorine, chlorine, methoxy, CN,
X⁶ is H, methyl, trifluoromethyl, difluoromethyl, difluoromethoxy, trifluoromethoxy, fluorine, methoxy, CN,
R¹ is H, methyl, ethyl, i-propyl, i-butyl,
R² is methyl, ethyl.

3. Process according to either of Claims 1 and 2, wherein the definitions of the radicals in the compounds of the general formulae (I), (II) and (III) are as follows:
X² is H,
X³ is H, methyl, trifluoromethyl, difluoromethyl, fluorine, chlorine, methoxy, CN,
X⁴ is fluorine, H,
X⁵ is H, methyl, trifluoromethyl, difluoromethyl, fluorine, chlorine, methoxy, CN,
X⁶ is H,
R¹ is H, methyl, *i*-propyl, *i*-butyl,
R² is methyl.

4. Process according to any of Claims 1 to 3, wherein the definitions of the radicals in the compounds of the general formulae (I), (II) and (III) are as follows:
X² is H,
X³ is H, fluorine,
X⁴ is H, fluorine,
X⁵ is H, fluorine,
X⁶ is H,
R¹ is H, methyl, *i*-butyl,
R² is methyl.

5. Process according to any of Claims 1 to 4, wherein the definitions of the radicals in the compounds of the general formulae (I), (II) and (III) are as follows:
X² is H,
X³ is fluorine,
X⁴ is H,
X⁵ is fluorine,
X⁶ is H,
R¹ is H, methyl,
R² is methyl.

6. Process according to any of Claims 1 to 5, wherein the definitions of the radicals in the compounds of the general formula (IIa) are as follows:
X⁷, X⁸, X¹⁰, X¹¹ are independently H, methyl, ethyl,
X⁹ is H, methyl, ethyl or N(methyl)₂.

7. Process according to any of Claims 1 to 6, wherein the definitions of the radicals in the compounds of the general formula (IIa) are as follows:
X⁷, X¹¹ is H,
X⁸, X¹⁰ are independently H, methyl, ethyl,
X⁹ is H, methyl, ethyl, N(methyl)₂.

8. Process according to any of Claims 1 to 7, wherein the definitions of the radicals in the compounds of the general formula (IIa) are as follows:
X⁸ is H, methyl, ethyl,
X⁷, X¹¹ is independently H, methyl,
X⁹, X¹⁰ is H.

9. Process according to any of Claims 1 to 8, wherein the compound of the general formula (IV) is generated by one of the following combinations of reagents:
- R⁴MgHal and R³OH or
- MgHal₂ and R³OM or
- MgHal₂ and Mg(OR³)₂, where
R³ is C₂-C₆-alkyl, benzyl, methylbenzyl,
Hal is halogen,
M is alkali metal,
R⁴ is C₁-C₆-alkyl, aryl, benzyl, allyl, vinyl.

10. Process according to any of Claims 1 to 9, wherein the compound of the general formula (IV) is generated by one of the following combinations of reagents:
- R⁴MgHal and R³OH or
- MgHal₂ and R³OM or
- MgHal₂ and Mg(OR³)₂, where
- R³ is C₂-C₄-alkyl,
- Hal is bromine or chlorine,
- M is alkali metal,
- R⁴ is C₁-C₄-alkyl, phenyl, benzyl, p-tolyl, vinyl.

11. Process according to any of Claims 1 to 10, wherein the compound of the general formula (IV) is generated by one of the following combinations of reagents:
- R⁴MgHal and R³OH or
- MgHal₂ and R³OM or
- MgHal₂ and Mg(OR³)₂, where
R³ is *i*-propyl, *i*-butyl,
Hal is bromine, chlorine,
M is sodium,
R⁴ is methyl, ethyl, *n*-butyl, *i*-propyl.

12. Process according to any of Claims 1 to 11, **characterized in that** 2.8 to 3.2 equivalents of the reactive species "R³OMgHal" (IV) are used, based on compounds of the general formula (II).

13. Process according to any of Claims 1 to 12, **characterized in that** the solvent is toluene, xylene, tetrahydrofuran (THF), isopropyl acetate (*i*-PrOAc), acetonitrile, methyl *tert*-butyl ether (MTBE), methyl-THF, ethyl acetate (EtOAc) or mixtures in any ratios thereof.

14. Process according to any of Claims 1 to 13, **characterized in that** the reaction is conducted at -25°C to 70°C.

15. Process according to any of Claims 1 to 13, **characterized in that** the reaction is conducted at 10°C to 30°C.

16. Process according to any of Claims 1 to 15, **characterized in that** the diastereomeric ratio is increased by a further crystallization step.

17. Process according to any of Claims 1 to 8, **characterized in that** the compounds of the general formula (IV) are generated via a Grignard reaction, with the following combinations of reagents:
R⁵MgHal and R⁶R⁷CO, where
R⁵ is C₁-C₆-alkyl, aryl, benzyl,
R⁶, R⁷ are H, C₁-C₆-alkyl, aryl, and the resulting radical definition
R³ is R⁵R⁶R⁷C.

18. Compounds of the formula (Ia) and (Ib) in any mixing ratio except 1:1, wherein the radicals have the definitions according to Claim 5, excluding methyl 3-(3,5-difluorophenyl)-5-(1-hydroxyethyl)-4H-isoxazole-5-carboxylate:

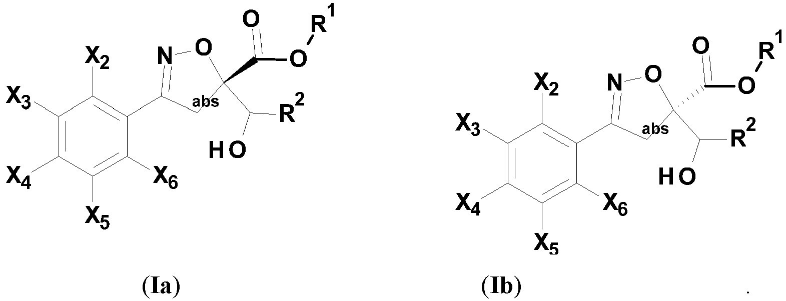

19. Compounds of the formula (Iaa) and (Iba) in any mixing ratio except 1:1, wherein the radicals have the definitions according to Claim 5, excluding methyl 3-(3,5-difluorophenyl)-5-(1-hydroxyethyl)-4H-isoxazole-5-carboxylate:

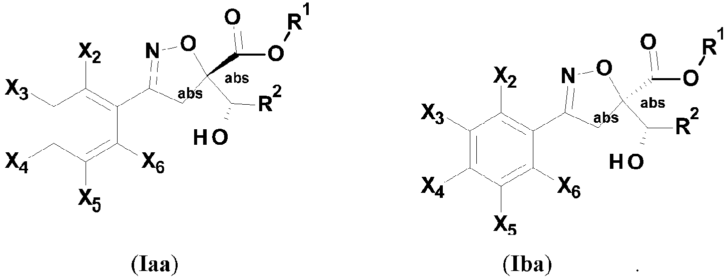

20. Compounds of the formula (Iab) and (Ibb) in any mixing ratio except 1:1, wherein the radicals have the definitions according Claim 5, excluding methyl 3-(3,5-difluorophenyl)-5-(1-hydroxyethyl)-4H-isoxazole-5-carboxylate:

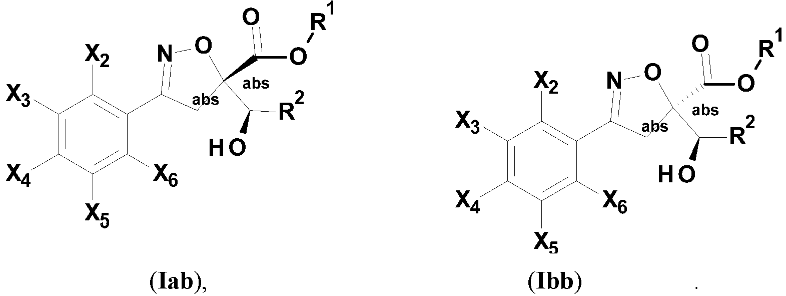

## Revendications

1. Procédé de préparation de dérivés d'acide isoxazolinecarboxylique de formule (I) 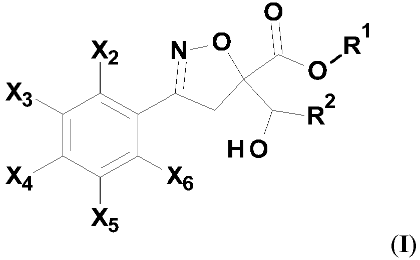 dans laquelle
X² représente H, C₁-C₄-alkyle, C₁-C₄-fluoroalkyle, C₁-C₄-fluoroalcoxy, C₁-C₄-alcoxy, fluor, CN,
X³ représente H, C₁-C₄-alkyle, C₁-C₄-fluoroalkyle, C₁-C₄-fluoroalcoxy, C₁-C₄-alcoxy, fluor, chlore, CN,
X⁴ représente H, C₁-C₄-alkyle, C₁-C₄-fluoroalkyle, C₁-C₄-fluoroalcoxy, C₁-C₄-alcoxy, fluor, CN,
X⁵ représente H, C₁-C₄-alkyle, C₁-C₄-fluoroalkyle, C₁-C₄-fluoroalcoxy, C₁-C₄-alcoxy, fluor, chlore, CN,
X⁶ représente H, C₁-C₄-alkyle, C₁-C₄-fluoroalkyle, C₁-C₄-fluoroalcoxy, C₁-C₄-alcoxy, fluor, CN,
R¹ représente H, C₁-C₄-alkyle,
R² représente C₁-C₄-alkyle,
**caractérisé en ce que** les composés de formule générale (II) 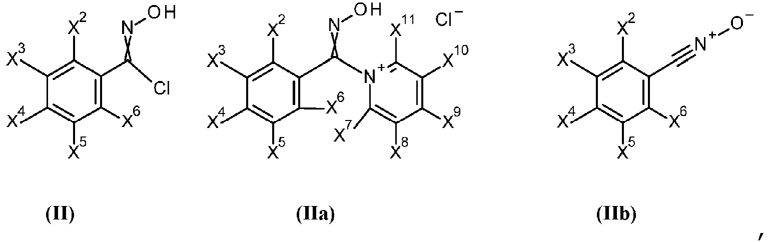
dans laquelle
X² à X⁶ présentent les significations susmentionnées, X⁷, X⁸, X¹⁰, X¹¹ signifient, indépendamment les uns des autres, H ou C₁-C₄-alkyle,
X⁹ signifie H, C₁-C₄-alkyle ou N(C₁-C₄-alkyle)₂,
sont transformés avec des composés de formule (III) 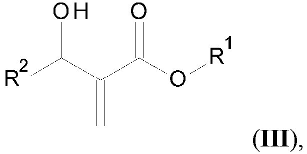
dans laquelle
R¹ et R² présentent les significations susmentionnées, avec ajout d'une combinaison de réactifs qui permet que - par rapport aux composés de formule générale (II) - 2,0 à 4,5 équivalents d'une espèce réactive "R³OMgHal" (IV), peuvent être formés
R³ signifiant C₂-C₆-alkyle, benzyle non substitué ou substitué par C₁-C₆-alkyle et
Hal représentant halogène,
en composés de formule générale (I).

2. Procédé selon la revendication 1, les définitions des radicaux pour les composés des formules générales (I), (II) et (III) étant les suivantes :
X² représente H, méthyle, trifluorométhyle, difluorométhyle, difluorométhoxy, trifluorométhoxy, fluor, méthoxy, CN,
X³ représente H, méthyle, trifluorométhyle, difluorométhyle, difluorométhoxy, trifluorométhoxy, fluor, chlore, méthoxy, CN,
X⁴ représente H, méthyle, trifluorométhyle, difluorométhyle, difluorométhoxy, trifluorométhoxy, fluor, méthoxy, CN,
X⁵ représente H, méthyle, trifluorométhyle, difluorométhyle, difluorométhoxy, trifluorométhoxy, fluor, chlore, méthoxy, CN,
X⁶ représente H, méthyle, trifluorométhyle, difluorométhyle, difluorométhoxy, trifluorométhoxy, fluor, méthoxy, CN,
R¹ représente H, méthyle, éthyle, i-propyle, *i*-butyle, R² représente méthyle, éthyle.

3. Procédé selon l'une des revendications 1 à 2, les définitions des radicaux pour les composés des formules générales (I), (II) et (III) étant les suivantes :
X² représente H,
X³ représente H, méthyle, trifluorométhyle, difluorométhyle, fluor, chlore, méthoxy, CN,
X⁴ représente fluor, H,
X⁵ représente H, méthyle, trifluorométhyle, difluorométhyle, fluor, chlore, méthoxy, CN,
X⁶ représente H,
R¹ représente H, méthyle, *i*-propyle, *i*-butyle,
R² représente méthyle.

4. Procédé selon l'une des revendications 1 à 3, les définitions des radicaux pour les composés des formules générales (I), (II) et (III) étant les suivantes :
X² représente H,
X³ représente H, fluor,
X⁴ représente H, fluor,
X⁵ représente H, fluor,
X⁶ représente H,
R¹ représente H, méthyle, *i*-butyle,
R² représente méthyle.

5. Procédé selon l'une des revendications 1 à 4, les définitions des radicaux pour les composés des formules générales (I), (II) et (III) étant les suivantes :
X² représente H,
X³ représente fluor,
X⁴ représente H,
X⁵ représente fluor,
X⁶ représente H,
R¹ représente H, méthyle,
R² représente méthyle.

6. Procédé selon l'une des revendications 1 à 5, les définitions des radicaux pour les composés de formule générale (IIa) étant les suivantes :
X⁷, X⁸, X¹⁰, X¹¹ signifient, indépendamment les uns des autres, H, méthyle, éthyle,
X⁹ signifie H, méthyle, éthyle ou N(méthyle)₂.

7. Procédé selon l'une des revendications 1 à 6, les définitions des radicaux pour les composés de formule générale (IIa) étant les suivantes :
X⁷, X¹¹ représentent H,
X⁸, X¹⁰ signifient, indépendamment l'un de l'autre, H, méthyle, éthyle,
X⁹ représente H, méthyle, éthyle, N(méthyle)₂.

8. Procédé selon l'une des revendications 1 à 7, les définitions des radicaux pour les composés de formule générale (IIa) étant les suivantes :
X⁸ représente H, méthyle, éthyle,
X⁷, X¹¹ signifient, indépendamment l'un de l'autre, H, méthyle,
X⁹, X¹⁰ représentent H.

9. Procédé selon l'une des revendications 1 à 8, le composé de formule générale (IV) étant généré à l'aide d'une des combinaisons de réactifs suivantes :
- R⁴MgHal et R³OH ou
- MgHal₂ et R³OM ou
- MgHal₂ et Mg(OR³)₂,
R³ signifiant C₂-C₆-alkyle, benzyle, méthylbenzyle,
Hal signifiant halogène,
M signifiant métal alcalin,
R⁴ signifiant C₁-C₆-alkyle, aryle, benzyle, allyle, vinyle.

10. Procédé selon l'une des revendications 1 à 9, le composé de formule générale (IV) étant généré à l'aide d'une des combinaisons de réactifs suivantes :
- R⁴MgHal et R³OH ou
- MgHal₂ et R³OM ou
- MgHal₂ et Mg(OR³)₂,
- R³ signifiant C₂-C₄-alkyle,
- Hal signifiant brome ou chlore,
- M signifiant métal alcalin,
- R⁴ signifiant C₁-C₄-alkyle, phényle, benzyle, p-toluyle, vinyle.

11. Procédé selon l'une des revendications 1 à 10, le composé de formule générale (IV) étant généré à l'aide d'une des combinaisons de réactifs suivantes :
- R⁴MgHal et R³OH ou
- MgHal₂ et R³OM ou
- MgHal₂ et Mg(OR³)₂,
- R³ signifiant *i*-propyle, *i*-butyle,
Hal signifiant brome, chlore,
M signifiant sodium,
R⁴ signifiant méthyle, éthyle, *n*-butyle, *i*-propyle.

12. Procédé selon l'une des revendications 1 à 11, **caractérisé en ce qu'**on utilise 2,8 à 3,2 équivalents de l'espace réactive "R³OMgHal" (IV) par rapport aux composés de formule générale (II).

13. Procédé selon l'une des revendications 1 à 12, **caractérisé en ce que** le solvant est le toluène, le xylène, le tétrahydrofuranne (THF), l'acétate d'isopropyle (*i*-PrOAc), l'acétonitrile, le méthyl-*tert-*butyléther (MTBE), le méthyl-THF, l'acétate d'éthyle (EtOAc) ou les mélanges dans différents rapports de ceux-ci.

14. Procédé selon l'une des revendications 1 à 13, **caractérisé en ce que** la réaction est réalisée à -25°C jusqu'à 70°C.

15. Procédé selon l'une des revendications 1 à 13, **caractérisé en ce que** la réaction est réalisée à 10°C jusqu'à 30°C.

16. Procédé selon l'une des revendications 1 à 15, **caractérisé en ce que** le rapport des diastéréoisomères peut être augmenté par une étape de cristallisation supplémentaire.

17. Procédé selon l'une des revendications 1 à 8, **caractérisé en ce que** les composés de formule générale (IV) sont générés par l'intermédiaire d'une réaction de Grignard et ce à l'aide des combinaisons de réactifs suivantes :
R⁵MgHal et R⁶R⁷CO,
R⁵ signifiant C₁-C₆-alkyle, aryle, benzyle, R⁶, R⁷ signifiant H, C₁-C₆-alkyle, aryle et la définition résultante du radical
R³ étant R⁵R⁶R⁷C.

18. Composés des formules (Ia) et (Ib), dans un rapport de mélange quelconque, à l'exception de 1:1, les radicaux présentant les définitions selon la revendication 5, à l'exception de l'ester méthylique de l'acide 3-(3,5-difluorophényl)-5-(1-hydroxyéthyl)-4H-isoxazole-5-carboxylique.

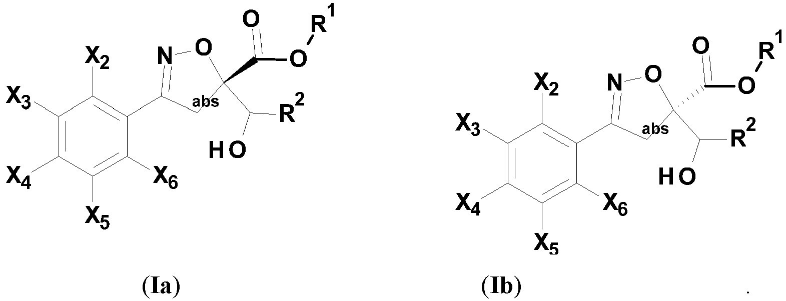

19. Composés des formules (Iaa) et (Iba), dans un rapport de mélange quelconque, à l'exception de 1:1, les radicaux présentant les définitions selon la revendication 5, à l'exception de l'ester méthylique de l'acide 3-(3,5-difluorophényl)-5-(1-hydroxyéthyl)-4H-isoxazole-5-carboxylique.

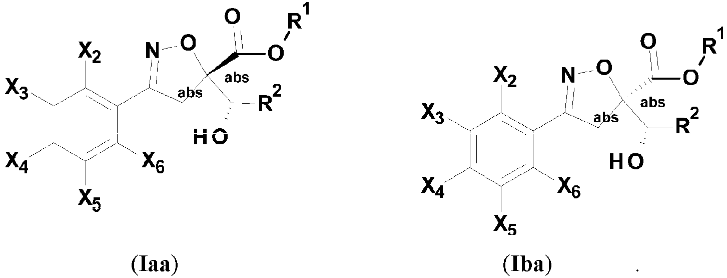

20. Composés des formules (Iab) et (Ibb), dans un rapport de mélange quelconque, à l'exception de 1:1, les radicaux présentant les définitions selon la revendication 5, à l'exception de l'ester méthylique de l'acide 3-(3,5-difluorophényl)-5-(1-hydroxyéthyl)-4H-isoxazole-5-carboxylique.
